# EUROPÄISCHE PATENTSCHRIFT

(11) **EP 1 286 937 B1**
(45) Veröffentlichungstag und Bekanntmachung des Hinweises auf die Patenterteilung: **14.03.2007**
(21) Anmeldenummer: 01957825.1
(22) Anmeldetag: 05.06.2001
(51) Int. Cl.: C07B 53/00, C07F 9/6574, C07F 9/6571, C07F 15/00, C07C 253/10

(54) **CHIRALE MONOPHOSPHITE ALS LIGANDEN FÜR ASYMMETRISCHE HYDRIERUNG**
CHIRAL MONOPHOSPHITES AS LIGANDS FOR ASYMETRICAL HDROGENATION
MONOPHOSPHITES CHIRAUX UTILISES COMME LIGANDS DANS LE HYDROGENATION ASYMETRIQUE

(30) Priorität: 06.06.2000 DE 10027505
(43) Veröffentlichungstag der Anmeldung: 05.03.2003
(73) Patentinhaber: Studiengesellschaft Kohle mbH, 45470 Mülheim (DE)
(72) Erfinder: REETZ, Manfred, Theodor, 45470 Mülheim/Ruhr (DE); MEHLER, Gerlinde, 45470 Mülheim/Ruhr (DE); MEISWINKEL, Andreas, 45470 Mühlheim/Ruhr (DE)
(74) Vertreter: Christophersen, Ruth
(86) Internationale Anmeldenummer: PCT/EP2001/006344
(87) Internationale Veröffentlichungsnummer: WO 2001/094278

(56) Entgegenhaltungen:
- WO-A-93/03839
- WO-A-95/29153
- PAMIES O. ET AL.: "Synthesis and coordination chemistry of novel chiral P,S-ligands with a xylofuranose backbone: use in asymmetric hydroformylation and hydrogenation" ORGANOMETALLICS., Bd. 19, Nr. 8, 2000, Seiten 1488-1496, XP001024882 ACS, COLUMBUS, OH., US ISSN: 0276-7333
- BAKER M.J. ET AL.: "Chiral aryl diphosphites: a new class of ligands for hydrocyanation catalysis" CHEMICAL COMMUNICATIONS., Nr. 18, 1991, Seiten 1292-1293, XP002180130 ROYAL SOCIETY OF CHEMISTRY., GB ISSN: 1359-7345
- REETZ M.T. ET AL.: "Highly enantioselective Rh-catalyzed hydrogenation reactions based on chiral monophosphite ligands" ANGEWANDTE CHEMIE. INTERNATIONAL EDITION., Bd. 39, Nr. 21, 3. November 2000 (2000-11-03), Seiten 3889-3890, XP002180131 VERLAG CHEMIE. WEINHEIM., DE ISSN: 0570-0833

## Beschreibung

Die vorliegende Erfindung beinhaltet den überraschenden Befund, dass bestimmte chirale Monophosphite und deren Monothioderivate ausgezeichnete Liganden bei der asymmetrischen Übergangsmetall-katalysierten Hydrierung von prochiralen Olefinen, Ketonen und Iminen darstellen.

Die katalytische enantioselektive Synthese hat in den letzten 20 Jahren industriell an Bedeutung gewonnen, so z. B. die Übergangsmetall-katalysierte asymmetrische Hydrierung (B. Cornils, W. A. Herrmann, *Applied Homogeneous Catalysis with Organometallic Compounds,* Wiley-VCH, Weinheim, **1996;** R. Noyori, *Asymmetric Catalysis in Organic Synthesis,* Wiley, New York, **1994**). Als Katalysatoren werden gewöhnlich Rhodium-, Ruthenium- oder Iridium-Komplexe von optisch aktiven Diphosphanen wie BINAP (R. Noyori *et al., J. Am. Chem. Soc.* **1980**, *102,* 7932), DuPHOS (M. J. *Burk et al., J. Am. Chem. Soc.* **1995**, 117, 9375), BICP (X. Zhang *et al., J. Am. Chem. Soc.* **1997,** 119, 1799) und BPE (M. J. Burk *et al., J. Am. Chem. Soc.,* **1996**, 118, 5142) verwendet. Nachteilig bei diesen Systemen ist der relative hohe präparative Aufwand bei der Darstellung und, ggf., der Antipodentrennung der racemischen Liganden sowie die oftmals unzureichende Enantioselektivität, die bei der Katalyse beobachtet wird. Darüber hinaus sind Phosphane oxidationsempfindlich, d. h. sie reagieren relativ leicht mit Luftsauerstoff, was zu einer Zerstörung der Liganden führt. Es ist daher das Ziel der industriellen und akademischen Forschung, neue und besonders leistungsfähige Liganden auf möglichst einfachem Weg herzustellen.

Auch bestimmte chirale Diphosphinite (T. V. RajanBabu, et. al., *J. Org. Chem.* **1997**, *62*, 6012) und Diphosphonite (M. T. Reetz, A. Gosberg, R. Goddard, S.-H. Kyung, *Chem. Commun. (Cambridge)* **1998**, 2077) wurden als Liganden in der Übergangsmetall-katalysierten Hydrierung eingesetzt. Dagegen ist nur wenig bekannt über chirale Diphosphite als Liganden bei diesen Reaktionen. Einige aus Kohlenhydraten oder Weinsäure abgeleitete Diphosphite führten bei der Rh-katalysierten Hydrierung von prochiralen Olefinen zu schlechten Enantioselektivitäten (*ee* = 1 - 34%) (H. Brunner *et al., J. Chem. Res. Synop.* **1980,** 76; D. J. Wink *et al., Inorg. Chem.* **1990,** 29, 5006). Dies ist bedauerlich, denn Phosphite sind bekanntlich deutlich weniger oxidationsempfindlich als Phosphane und müssen infolgedessen nicht ständig unter Luftausschluss, d. h. unter Inertgasbedingungen, gehandhabt werden. Deshalb erschien der Bericht interessant, dass bestimmte aus Dianhydro-D-mannit abgeleitete chirale Diphosphite bei der Rh-katalysierten Hydrierung von prochiralen Olefinen z. T. sehr hohe Enantioselektivitäten liefern (ee bis zu 98%) (M. T. Reetz, T. Neugebauer, *Angew. Chem.* **1999,** *111*, **134;** *Angew. Chem., Int. Ed.* **1999,** *38*, 179). Nachteilig ist jedoch der Befund, dass eine so hohe Enantioselektivität die Ausnahme ist, und insbesondere, dass das Dianhydro-D-mannit verhältnismäßig schwer zugänglich und infolgedessen teuer ist.

In Organometallics, 2000, Vol. 19 (8), 1488-1496 werden Übergangsmetall-katalysatoren offenbart, die P,S-Liganden mit einem Xylofuranose-Rückgrat aufweisen. Im Molekül der Liganden ist mindestens eine Bindungsgruppe am Phosphor ein von Kohlenhydraten abgeleiteter Ring gebunden, nämlich Xylofuranose.Die Katalysatoren werden in der asymmetrischen Hydroformylierung und Hydrierung eingesetzt, wobei Enantioselektivitäten von nur 51 % und 47 % erhalten werden.

Aus J. Chem. Soc., Chem. Commun. 1991, 1292-93 sind Nickel-Katalysatoren mit organischen Ligenden bekannt, die zur Hydrocyanierung von Norbornen eingesetzt werden.

Diese Nachteile treffen nicht zu auf die vorliegende Erfindung, in der chirale Monophosphite und deren Monothioderivate als Liganden in der asymmetrischen Hydrierung von prochiralen Olefinen, Ketonen und Iminen verwendet werden.
Die Synthese dieser chiralen Liganden ist in mehreren Publikationen beschrieben (s. z.B. US5962744A; WO9529153A1; WO9303839A1; US4599206). Der modulare Aufbau erlaubt es, eine Vielzahl von neuen chiralen Monophosphiten in enantiomerenreiner Form herzustellen.
Jedoch hat die Verwendung von chiralen Monophosphiten als Liganden in der metallkatalysierten Hydroformylierung (s. z.B. WO9529153A1), Hydrosilylierung (s. z.B. US5360938; WO9303839A1), Cyclopropanierung (s. z.B. US5360938; WO9303839A1) und Aldol-Addition (s. z.B. US5360938; WO9303839A1) in keinem Fall zu merklichen Enantioselektivitäten geführt (ee = 10-26%). Über die Verwendung von chiralen Monophosphiten bei Hydrierungen ist nichts bekannt. Zwar gibt es Spekulationen darüber (US5360938; WO9303839A1), die jedoch durch keine Experimente belegt sind. Darüberhinaus liegen die dort erzielten *ee-*Werte bei der Hydrosilylierung, Cyclopropanierung und Aldoladdition in allen Fällen unter 26%. Die Fachwelt ist davon ausgegangen, dass bidentate bzw. chelatisierende Liganden erforderlich sind, die Freiheitsgrade einschränken, um hohe und industriell nützliche Enantioselektivitäten zu ermöglichen (s. z.B. I. V. Komarov, A. Börner, *Angew. Chem.* **2001,** *113,* 1237; *Angew. Chem. Int. Ed* **2001,** *40,* 1197; E. N. Jacobsen, A. Pfaltz, H. Yamamoti, *Comprehensive Asymmetric Catalysis,* Springer, Berlin, **1999**).

Während die eingangs beschriebenen chiralen Diphosphite und die "Standardliganden" von Typ BINAP, DuPHOS und PennPHOS (E. N. Jacobsen, A. Pfaltz, H. Yamamoti, *Comprehensive Asymmetric Catalysis,* Springer, Berlin, 1999) sowie gemischte Liganden wie Phosphan-phosphite oder Phosphonit-phosphite (M. T. Reetz, M. Pastó, *Tetrahedron Lett.* **2000,** *41,* 3315) chelatisierend sind und aufgrund dieser Eigenschaft hohe Enantioselektivitäten liefern, sind die Liganden der vorliegenden Erfindung keine chelatisierenden Verbindungen mit zwei Donorstellen.
Es wurde nun überraschenderweise gefunden, dass genau die oben beschriebenen Monophosphite sowie die analogen Monothioderivate ausgezeichnete Liganden bei der asymmetrischen Übergangsmetall-katalysierten Hydrierung sind, die zu hohen Enantioselektivitäten führen. Die erzielten ee-Werte liegen sehr häufig über 90%. Die Liganden sind nicht nur leicht darstellbar, sondern verfügen auch über eine relativ hohe Oxidationsbeständigkeit.

Im einzelnen umfasst die Erfindung Monosphosphite (Y = O) sowie deren Monothioderivate (Y = S) des Typs **I** bis **IV** als Liganden.

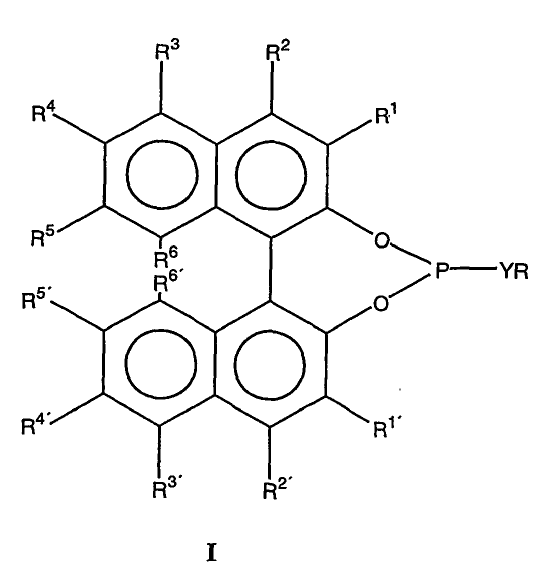

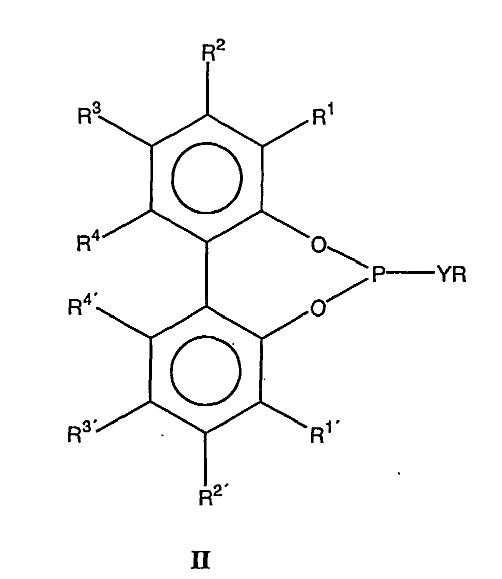

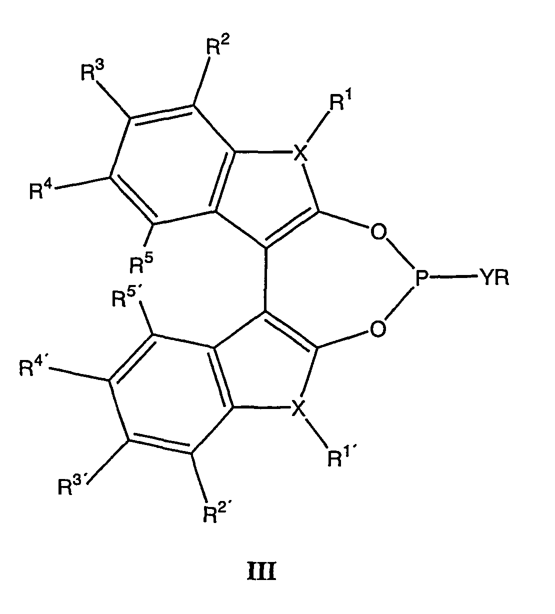

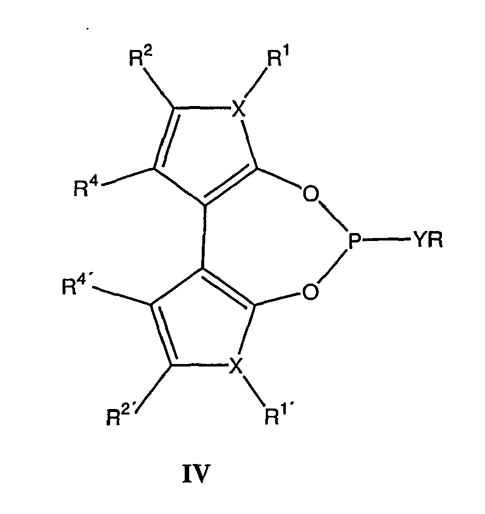

Im Falle der Ligandenklasse **I** enthalten die Verbindungen eine axial chirale Einheit, die sich von Binaphthyl ableitet, mit Resten R¹, R², R³, R⁴, R⁵, R⁶, R^{1'}, R^{2'}, R^{3'}, R^{4'}, R^{5'} und R^{6'}, die unabhängig voneinander nachfolgende Gruppen darstellen können: Wasserstoff (H), gesättigte Kohlenwasserstoffe, gegebenenfalls auch funktionalisiert und/oder verbrückend (z. B. R¹ + R²= -(CH₂)₄-), aromatische oder heteroaromatische Gruppen, die auch funktionalisiert und/oder anelliert sein können und somit cyclische Reste darstellen (beispielsweise R¹ + R² = R^{1'} + R²'= ortho-Phenylen; entspricht 4,4'-Dihydroxy-5,5'-bis(phenanthryl)), nichtaromatische ungesättigte Kohlenwasserstoffe wie z. B. Alkinylgruppen -C≡CR', die auch funktionalisiert sein können, Silylgruppen wie z. B. -SiMe₃, Halogene (-Cl, -Br, -F, -I), Nitro- (-NO₂ oder Nitrilgruppen (-CN), außerdem Ester (-CO₂R'), Carbonyle (-C(O)R'), Amide (-C(O)NR'R"), Amine (-NR'R"), Ether (-OR'), Alkoxy (-OR'), Sulfide (-SR') und Selenide (-SeR') in denen R' und R" Wasserstoff, gesättigte oder nichtaromatische ungesättigte Kohlenwasserstoffe, gegebenenfalls auch funktionalisiert, oder aromatische Reste, gegebenenfalls auch funktionalisiert, sind. Insbesondere sind in der vorgestellten Erfindung alle Kombinationen der genannten Reste für R¹, R², R³, R⁴, R⁵, R⁶, R^{1'}, R^{2'}, R^{3'}, R^{4'}, R^{5'} und R^{6'} einschließlich aller *C*₁- und C₂-symmetrischen Substitutionsmuster des Binaphthylgrundkörpers enthalten. Ferner können einzelne oder mehrere Kohlenstoffatome des Binaphthylgerüstes durch Heteroatome wie z. B. Si, 0, S oder N ersetzt sein. Vorzugsweise dient Binaphthyl (R¹ =R² = R³ = R⁴ = R⁵ = R⁶ = R^{1'} = R^{2'} = R^{3'} = R^{4'} = R^{5'} = R^{6'} = H) selbst als axial chirales Element.

Im Falle der Verbindungsklasse **II** ist die axial chirale Einheit ein konfigurativ stabiles Biphenylderivat. Konfigurative Stabilität hinsichtlich der axialen Chiralität ist dann gewährleistet, wenn R⁴ # H und R⁴' ≠ H (E. L. Eliel, S. H. Wilen, L. N. Mander, *Stereochemistry of Organic Compounds,* Wiley, New York, 1994). R¹ bis R⁴ und R^{1'} bis R^{4'} weisen hierbei dieselbe Variationsbreite wie die Reste R¹ bis R⁶ und R^{1'} bis R^{6'} im Falle der Verbindungsklasse **I** auf. Vorzugsweise ist jedoch R¹ = R² = R¹' = R^{2'} = H und R³ + R⁴ = R³' + R^{4'} = -(CH₂)₄- (entspricht 5,5'-6,6'-7,7'-8,8'-Octahydro-1,1'-binaphthyl, D. J. Cram *et al., J. Org. Chem.* **1978**, *43,* 1930). Auch in Liganden des Typs **II** können einzelne oder mehrere Kohlenstoffatome des Biphenylgerüstes durch Heteroatome wie z. B. Si, O, S oder N ersetzt sein

Im Falle der Verbindungsklasse **III** enthalten die Verbindungen eine axial chirale Einheit, die sich von 3,3'-Bis(indolyl) (X = N), 3,3'-Bis(benzo[b]thiophenyl) (X = S) oder 3,3'-Bis(benzo[b]furanyl) (X = O) ableitet. Auch in diesen Fällen weisen die Substituenten R¹ bis R⁵ und R^{1'} bis R⁵' dieselbe Variationsbreite auf wie die Reste R¹ bis R⁶ und R¹' bis R^{6*'*} im Falle der Verbindungsklasse **I**. Die Substituenten R¹ und R^{1'} entfallen für die Fälle X = O und X = S.

Im Falle der Verbindungsklasse **IV** enthalten die Verbindungen als axial chirale Einheit ein konfigurativ stabiles heteroaromatisches System, das sich von 3,3'-Bis(pyrrolyl) (X = N), 3,3'-Bis(thiophenyl) (X = S) oder 3,3'-Bis(furanyl) (X = O) ableitet und ebenfalls bekannt ist. Hinsichtlich der Reste R¹ R², R⁴, R^{1'}, R^{2'} und R^{4'} bestehen dieselben Variationsmöglichkeiten wie für die Reste R¹ bis R⁵ und R^{1'} bis R*^{5'}* der Verbindungsklasse **III** beschrieben.

Im Falle der Verbindungsklassen **II** und **IV** kann auch R⁴ = R⁴' = H sein. In diesem Fall erhält man ein konfigurativ labiles axiales Element und Chiralität wird dadurch erreicht, dass ein chiraler Rest R verwendet wird.

Die vorliegende Erfindung beinhaltet alle stereoisomeren Formen der Verbindungen **I - IV**.

Der Rest R bei den chiralen Phosphiten **I - IV** leitet sich aus achiralen oder chiralen Alkoholen, Phenolen oder Hydroxyheteroaromaten (Y = O) oder deren Schwefelanaloga (Y = S) ab (**V**), die gegebenenfalls jeweils zusätzliche funktionelle Gruppen wie z. B. Amino-, Ether-, Thioether-, Halogen- oder Ester-Reste tragen.

RYH V

Im einfachsten Fall handelt es sich um gängige Alkohole (Y = O) wie Methanol, Ethanol, Propanol oder Isopropanol usw., also um achirale Alkohole aus C₁ bis C₅₀ Bausteinen. Es kommen jedoch auch chirale Alkohole wie 1-Phenylethanol, Terpen-, Kohlenhydrat- oder Steroid-Alkohole in Frage. Einige wenige Beispiele für solche Monophosphite sind in der Literatur bekannt, so. z. B. I mit R¹ = R² = R³=R⁴=R⁵=R⁶=R^{1'}=R^{2'}=R^{3'}=R^{4'}=R^{5'}=R^{6'}=H und R=Menthyl (P. H. *Dussault et al., J. Org. Chem.* **1997**, 62, 1556). Funktionalisierte Alkohole wie z. B. β-Aminoalkohole oder α-Hydroxycarbonsäureester sind ebenfalls möglich. R kann auch einen aromatischen Rest darstellen, RYH (**V**) bedeutet in diesen Fällen Phenol-artige Verbindungen, typischerweise Phenol oder α- bzw. β-Naphthol bzw. substituierte Derivate wie z. B. p-, m-, o-, Alkyl-, Aryl-, Amino-, Chloro-, Bromo-, Cyano-Phenol. Verbindung **I** mit R¹ = R² = R³ = R⁴ = R⁵ = R⁶ = R^{1'} = R^{2'} = R^{3'} = R⁴' = R^{5'} = R^{6'} = H und R = Phenyl ist in der Literatur bekannt (K. Fuji *et al., Chem. Commun. (Cambridge)* **1999,** 2289). RYH (V) kann auch ein Hydroxy-Heteroaren darstellen, wie z. B. 2-, 3- und 4-Hydroxypyridin, 2-, 3-Hydroxyfuran oder 2-, 3-Hydroxythiophen.
Für die entsprechenden Schwefelverbindungen (Y = S) kommen achirale und chirale Thiole V in Betracht. Der Rest R kann hier genauso wie für die Alkohole beschrieben variiert werden.

Um die erfindungsgemäßen asymmetrischen Hydrierungen durchführen zu können, müssen erst Katalysatoren bzw. Präkatalysatoren durch Reaktion der chiralen Monophosphite mit geeigneten Übergangsmetallsalzen (insbesondere Metalle der Gruppen VIII und Ib des Periodensystems) hergestellt werden. Typische Vertreter sind z. B. die folgenden

Verbindungen, wobei cod für η²,η²-1,5-Cyclooctadien und cymol für η⁶-1-isoPropyl-4-methylbenzol stehen:

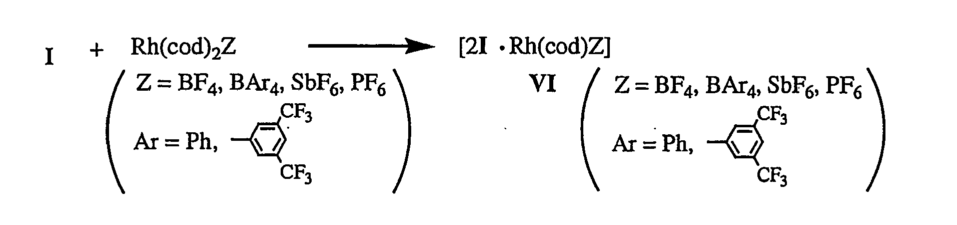

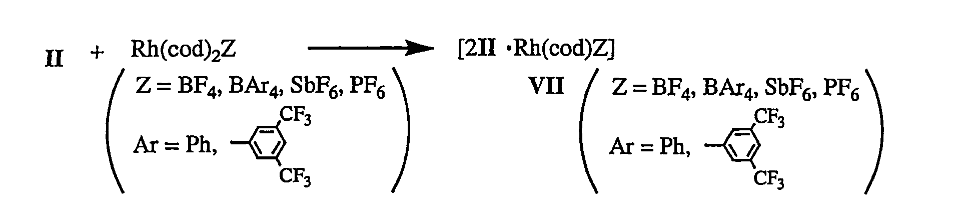

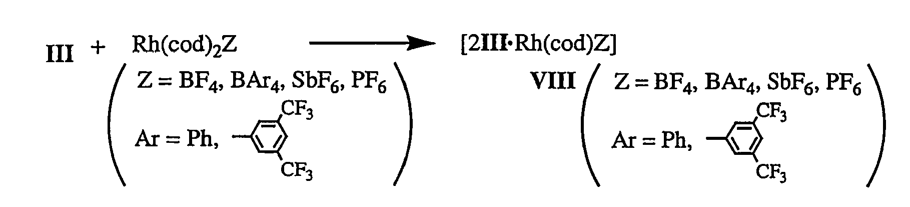

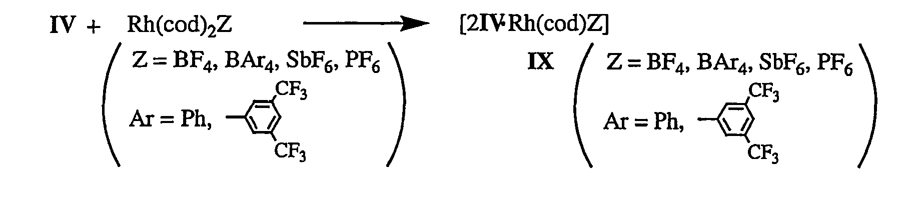

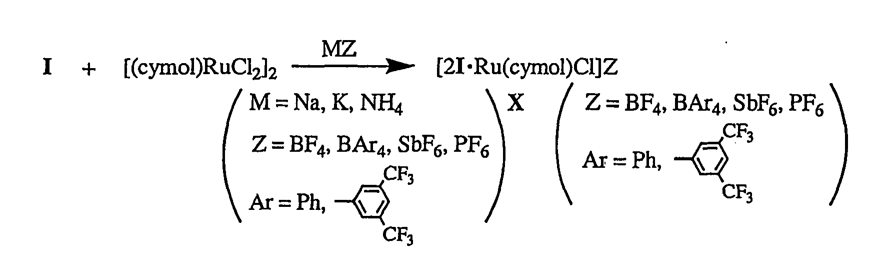

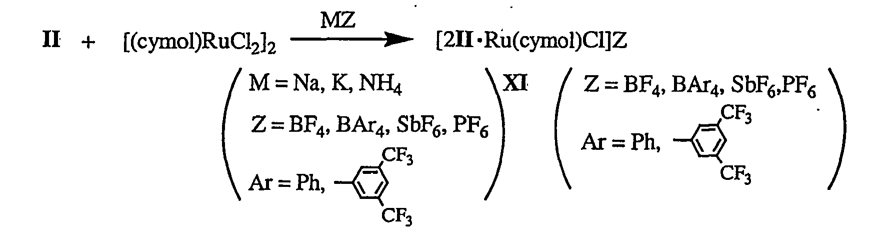

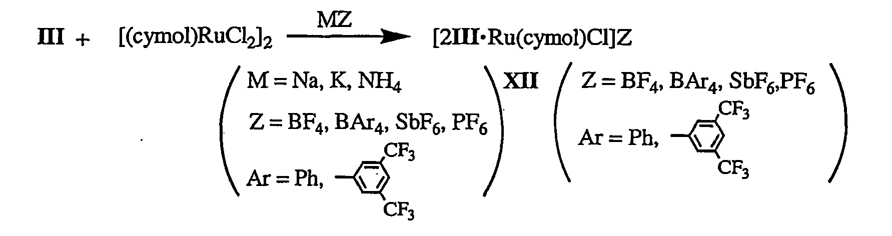

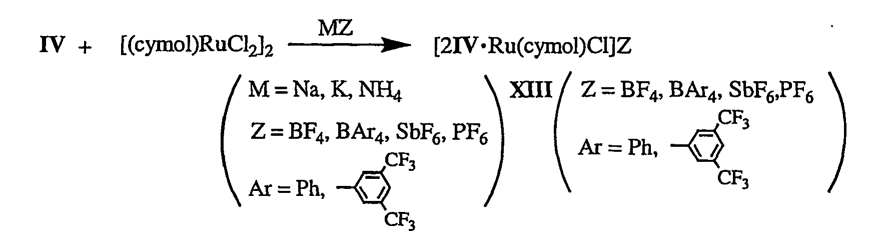

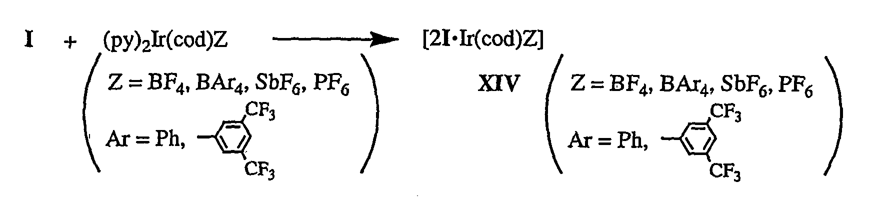

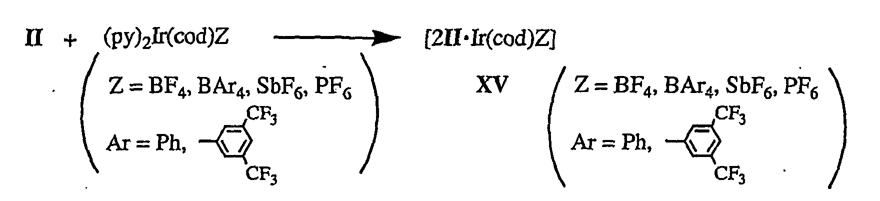

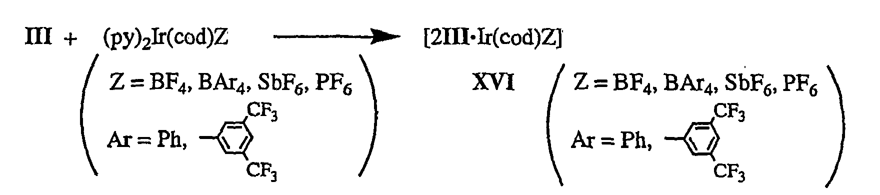

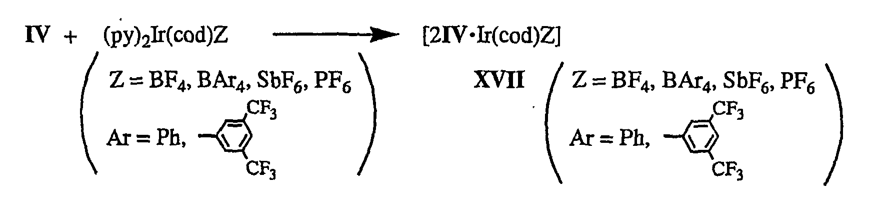

I + Ni(cod)₂ → [2I·Ni(cod)] XIIX

II + Ni(cod)₂ → [2II·Ni(cod)] XIX

III + Ni(cod)₂ → [2III·Ni(cod)] XX

IV + Ni(cod)₂ → [2IV·Ni(cod)] XXI

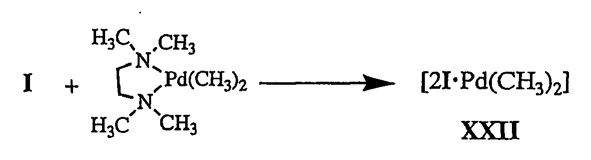

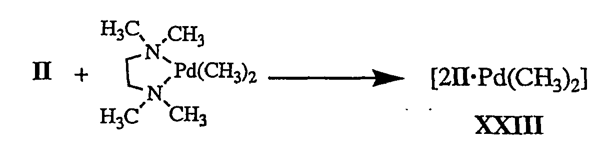

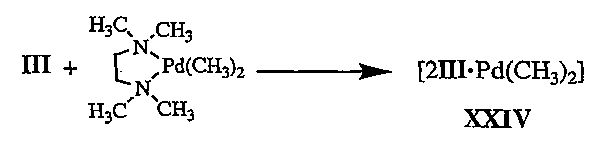

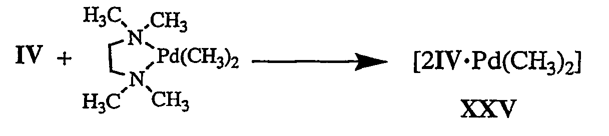

I + Pt(cod)₂ → [2I·Pt(cod)] XXV

II + Pt(cod)₂ → [2II·Pt(cod)] XXVII

III + Pt(cod)₂ → [2III·Pt(cod)] XXIIX

IV + Pt(cod)₂ → [2IV·Pt(cod)] XXIV

Diese Katalysatoren bzw. Präkatalysatoren sind nicht die einzigen Möglichkeiten, vielmehr kommen z. B. die in der Literatur üblichen kationischen Formen der Übergangsmetall-Verbindungen ebenfalls in Frage.

Die Erfindung beinhaltet also die Anwendung solcher Metallkomplexe in der asymmetrischen Hydrierung von prochiralen Olefinen, Ketonen und Iminen. Zur Illustration seien folgende Reaktionen als typische Beispiele erwähnt. Solche Produkte sind industriell wertvoll, so z. B. als Bausteine bei der Synthese von chiralen Wirkstoffen (Medikamente, Pflanzenschutzmittel usw.).

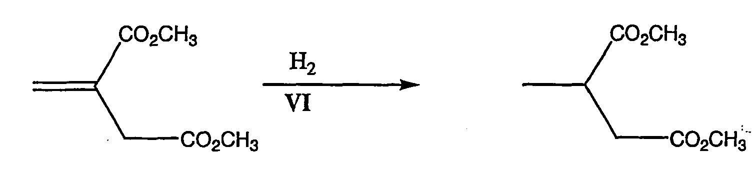

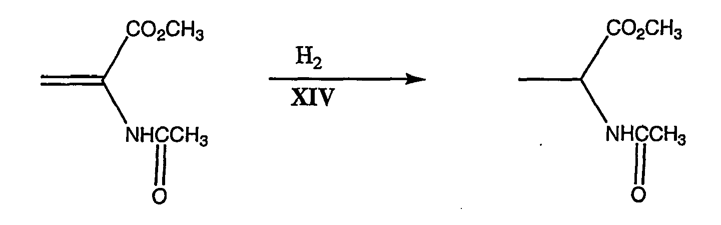

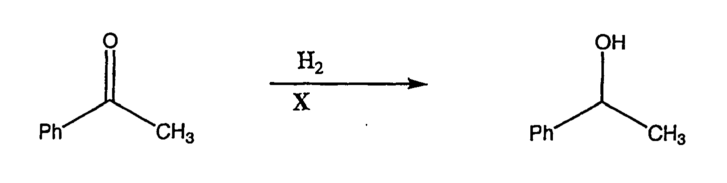

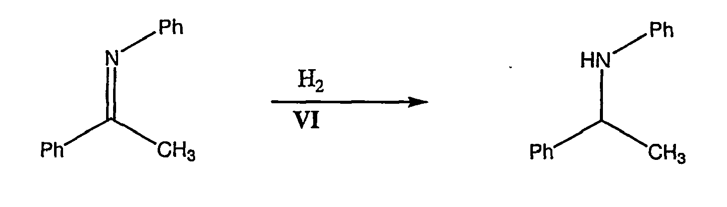

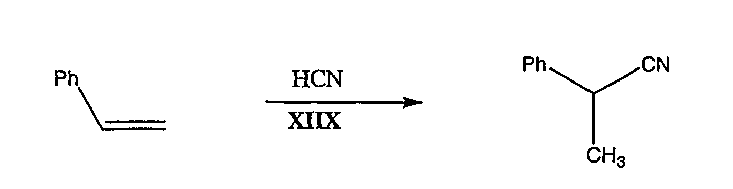

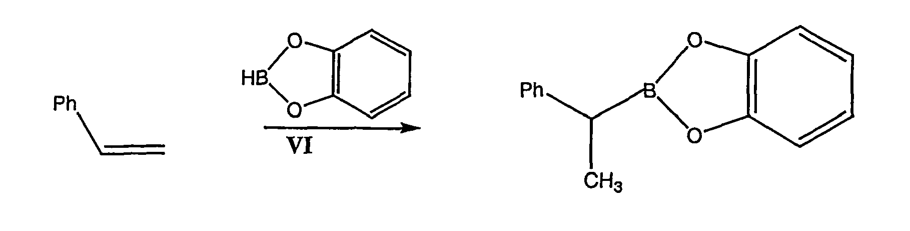

Die Erfindung wird mit folgenden Beispielen belegt, ohne sie dadurch zu beschränken:

### Darstellung von Monophosphitliganden

### Beispiel 1. Synthese von (S)-2,2'-Binaphthylphosphorigsäureisopropylester (I: R¹ = R² = R³ = R⁴ = R⁵ = R⁶ = R^{1'} = R^{2'} = R^{3'} = R^{4'} = R^{5'}= R^{6'} = H; Y = O; R = CH(CH₃)₂)

0.751 g (2.1 mmol) (*S*)-2,2'-Binaphthylphosphorigsäureesterchlorid wurden bei Raumtemperatur in 100 ml abs. Diethylether vorgelegt. Hierzu wurden 160 µl (0.126 g, 2.1 mmol) abs. Isopropanol und 0.29 ml (0.212 g, 2.3 mmol) abs. Triethylamin bei Raumtemperatur pipettiert. Nach Rühren über Nacht wurde der ausgefallene farblose Feststoff über eine D4-Fritte abfiltriert und mit 5 ml abs. Diethylether gewaschen. Das Filtrat wurde anschließend vollständig vom Lösungsmittel befreit. Man erhielt 0.60 g (1.6 mmol, 74.9%) Produkt als farbloses Pulver. Analytik: ¹H-NMR (CD₂Cl₂, 300 MHz): 7.88-7.12 [12H], 4.41 (m) [1H], 1.21 (d) *J* = 6.15 Hz [3H]; 1.16 (d) *J* = 6.18 Hz [3H]; ¹³C-NMR (CD₂Cl₂,75 Hz): 68.89 (d), *J* = 13.6 Hz; 23.70 (d) *J* = 3.2 Hz, 23.37 (d) *J* = 4.0 Hz; ³¹P-NMR (CD₂Cl₂, 121 MHz): 147.600(s); MS (EI, Verdampfungstemperatur 100°C): m/z = 374 (100%), 313 (22.5%), 239 (68.6%); EA: C: 73.21% (ber. 73.79%), H: 4.88% (ber. 5.11%),P: 8.67% (ber. 8.27%).

### Beispiel 2. Synthese von (S)-2,2'-Binaphthylphosphorigsäure[(S)-1-phenylethyl]ester (I: R¹ = R² = R³ = R⁴ = R⁵ = R⁶ = R^{1'} = R^{2'} = R^{3'} = R^{4'}= R^{5'} = R^{6'} = H; Y = O; R = (S)-1-Phenylethyl)

0.51 g (1.50 mmol) (*S*)-2,2'-Binaphthylphosphorigsäureesterchlorid wurden bei Raumtemperatur in 100 ml abs. Diethylether vorgelegt. Hierzu wurden 180 µl (0.183 g, 1.50 mmol) abs. (S)-(-)-1-Phenylethanol und 0.23 ml (0.167 g, 1.65 mmol) abs. Triethylamin bei Raumtemperatur pipettiert. Nach Rühren über Nacht wurde der ausgefallene farblose Feststoff über eine D4-Fritte abfiltriert und mit 5 ml abs. Diethylether gewaschen. Das Filtrat wurde anschließend vollständig vom Lösungsmittel befreit. Man erhielt 0.44 g (1.0 mmol, 66.6%) Produkt als farbloses Pulver. Analytik: ¹H-NMR (CD₂Cl₂, 300 MHz): 7.84 - 7.04 [17H], 5.27 (m) [1H], 1.44 (d) *J* = 6.5 Hz [3H]; ¹³C-NMR (CD₂Cl₂, 75 MHz): 65.25, 24.90 (d) J = 3.1 Hz; ³¹P-NMR (CD₂Cl₂, 121 MHz): 144.80(s); EA: C: 69.85% (ber. 77.05%), H: 5.95% (ber. 4.85%),P: 7.07% (ber. 7.09%).

### Beispiel 3. Synthese von (R)-2,2'-Binaphthylphosphorigsäureneopentylester (I: R¹=R²=R³=R⁴=R⁵=R⁶=R^{1'}=R^{2'}=R^{3'}=R^{4'}=R^{5'}=R^{6'}=H; Y=O; R= CH₂C(CH₃)₃)

0.67 g (1.90 mmol) (*R*)-2,2'-Binaphthylphosphorigsäureesterchlorid wurden bei Raumtemperatur in 100 ml abs. Toluol vorgelegt. Hierzu wurden 0.5 ml (0.36 g, 3.67 mmol) abs. Triethylamin und 0.17 g (1.90 mmol) Neopentanol bei Raumtemperatur gelöst in 15 ml abs. Toluol pipettiert. Nach Rühren über Nacht wurde der ausgefallene farblose Feststoff über eine D4-Fritte abfiltriert und mit 5 ml abs. Toluol gewaschen. Das Filtrat wurde anschließend vollständig vom Lösungsmittel befreit. Man erhielt 0.72 g (1.79 mmol, 94.1 %) Produkt als farbloses Pulver. Analytik: ¹H-NMR (CDCl₃, 300 MHz): 7.90-7.82 [4H], 7.45-7.14 [8H], 3.58 (m) [1H], 3.36 (m) [1H], 0.82 (s) [9H]; ³¹P-NMR (CDCl₃, 121 MHz): 143.395 (s); MS (EI, Verdampfungstemperatur 110°C): m/z = 402 (73.49%), 332 (100%), 313 (18.69%), 268 (53.52%), 239 (28.97%); EA: C: 74.56% (ber. 74.62%), H: 5.72% (ber. 5.76%), P: 7.68% (ber.7.70%).

### Beispiel 4. Synthese von (S)-2,2'-Binaphthylphosphorigsäure-(3-N,N-dimethylamino)-phenylester (I: R¹ = R² = R³ = R⁴ = R⁵ = R⁶ = R^{1'} = R^{2'} = R^{3'}= R^{4'}= R^{5'}= R⁶' = H; Y = O; R = C₆H₄N(CH₃)₂ = 3-N,N-Dimethylaminophenyl)

0.7 g (1.99 mmol) (S)-2,2'-Binaphthylphosphorigsäureesterchlorid wurden bei Raumtemperatur in 100 ml abs. Diethylether vorgelegt. Hierzu wurden 0.27 g (1.99 mmol) abs. 3-N,N-Dimethylaminophenol und 0.31 ml (0.22 g, 2,20 mmol) abs. Triethylamin bei Raumtemperatur pipettiert. Nach Rühren über Nacht wurde der ausgefallene farblose Feststoff über eine D4-Fritte abfiltriert und mit 5 ml abs. Diethylether gewaschen. Das Filtrat wurde anschließend vollständig vom Lösungsmittel befreit. Man erhielt 0.57 g (1.26 mmol, 63.5%) Produkt als farbloses Pulver. Analytik: ¹H-NMR (CD₂Cl₂, 300 MHz): 7.92-6.35 [16H], 2.72 (s) [6H]; ³¹P-NMR (CD₂Cl₂, 121 MHz): 145.38 (d), J = 85Hz; MS (EI, Verdampfungstemperatur 150°C): m/z = 451 (100%), 315 (22.6%), 268 (44.4%); EA: C: 73.84% (ber. 74.49%), H: 5.68% (ber. 4.91%), N: 3.45% (ber. 3.10%). P: 6.17% (ber. 6.86%).

### Beispiel 5. Synthese von (S)-2,2'-Binaphthylphosphorigsäure-(N,N-Di-iso-propylamino)-ethylester (I: R¹ = R² = R³ = R⁴ = R⁵ = R⁶ = R^{1'} = R^{2'} = R^{3'} = R^{4'} = R⁵' = R^{6'} = H; Y = O; R = (CH₂)₂N(CH(CH₃)₂)₂ = N,N-Di-iso-propylaminoethyl);

0.7 g (1.99 mmol) (S)-2,2'-Binaphthylphosphorigsäureesterchlorid werden bei Raumtemperatur in 100 ml abs. Diethylether vorgelegt. Hierzu wurden 0.35 ml (0.29 g, 1.99 mmol) abs. (N,N-Di-*iso*-propylamino)-ethanol und 0.31 ml (0.22 g, 2.20 mmol) abs. Triethylamin bei Raumtemperatur pipettiert. Nach Rühren über Nacht wurde der ausgefallene farblose Feststoff über eine D4-Fritte abfiltriert und mit 5 ml abs. Diethylether gewaschen. Das Filtrat wurde anschließend vollständig vom Lösungsmittel befreit. Man erhielt 0.52 g (1.13 mmol, 56.6%) Produkt als farbloses Pulver. Analytik: ¹H-NMR (CD₂Cl₂, 300 MHz): 7.89-7.10 [12H], 3.63(m) [2H], 2.79 (m) [2H], 2.51(t) [2H] J = 7.3Hz, 0.81 (d) [6H] J = 4.9Hz, 0.78 (d) [6H] J = 4.9Hz ; ³¹P-NMR (CD₂Cl₂, 121 MHz): 144.344 (d), J = 85Hz; EA: C: 72.79% (ber. 73.18%), H: 6.51% (ber. 6.58%), N: 2.99% (ber. 3.04%), P: 6.83% (ber. 6.74%).

### Beispiel 6. (S)-2,2'-Binaphthylphosphorigsäure[(S)-2-ethylcarboxy-2-ethyl]ester (I: R¹ =R²=R³=R⁴=R⁵=R⁶=R^{1'}=R^{2'}=R^{3'}=R^{4'}=R^{5'}=R^{6'}=H; Y=O; R = (S)-CH(CH₃)CO₂CH₂CH₃)

0.62 g (1.77 mmol) (S)-2,2'-Binaphthylphosphorigsäureesterchlorid wurden bei Raumtemperatur in 100 ml abs. Toluol vorgelegt. Hierzu wurden 0.50 ml (0.36 g, 3.67 mmol) abs. Triethylamin und 0.20 ml (0.21 g, 1.77 mmol) (S)-Milchsäureethylester bei Raumtemperatur pipettiert. Nach Rühren über Nacht wurde der ausgefallene farblose Feststoff über eine D4-Fritte abfiltriert und mit 5 ml abs. Toluol gewaschen. Das Filtrat wurde anschließend vollständig vom Lösungsmittel befreit. Man erhielt 0.58 g (1.34 mmol, 75.8%) Produkt als farbloses Pulver. Analytik: ¹H-NMR (CDCl₃, 300 MHz): 7.92-7.80 [4H], 7.47-7.18 [8H], 4.62 (m) [1H], 4.16 (q) *J* = 7.1 Hz [2H], 1.42 (d) *J* = 6.9 Hz [3H], 1.21 (t) *J* = 7.1 Hz [3H]; ³¹P-NMR (CDCl₃, 121 MHz): 141.507 (s); MS (EI, Verdampfungstemperatur 130°C): m/z = 432 (87.41%), 331, (100%), 315 (31.30%), 268 (63.59%), 239 (31.11%); EA: C: 69.32% (ber. 69.44%), H: 4.04% (ber. 4.90%), P: 7.30% (ber. 7.16%).

### Beispiel 7. Synthese von (S)-2,2'-Binaphthylphosphorigsäure-(2-methoxy)ethylester (I: R¹=R²=R³=R⁴=R⁵=R⁶=R^{1'}=R^{2'}=R^{3'}=R^{4'}=R^{5'}=R^{6'}=H; Y= O; R = CH₂CH₂OCH₃)

1.43g (4.1 mmol) (*S*)-2,2'-Binaphthylphosphorigsäureesterchlorid wurden bei Raumtemperatur in 100 ml abs. Diethylether vorgelegt. Hierzu wurden 0:32 ml (0.31 g, 4.1 mmol) 2-Methoxyethanol und 0.59 ml (0.46 g, 4.50 mmol) abs. Triethylamin bei Raumtemperatur pipettiert. Nach Rühren über Nacht wurde der ausgefallene farblose Feststoff über eine D4-Fritte abfiltriert und mit 5 ml abs. Diethylether gewaschen. Das Filtrat wurde anschließend vollständig vom Lösungsmittel befreit. Man erhielt 0.92 g (2.35 mmol, 57.5%) Produkt als farbloses Pulver. Analytik: ¹H-NMR (CD₂Cl₂, 300 MHz): 7.87-7.08 [12H], 3.94 (m) [1H], 3.77 (m) [1H], 3.33(m) [2H], 3.21 (s) [3H]; ³¹P-NMR (CD₂Cl₂, 121 MHz): 145.734 (s); MS (EI, Verdampfungstemperatur 135°C): m/z = 390 (100%), 331 (88.47%), 268 (65.78%); EA: C: 69.88% (ber. 70.76%), H: 5.49% (ber. 4.90%), P: 7.51% (ber. 7.93%).

### Beispiel 8. Synthese von 2,2'-Biphenylphosphorigsäure[(S)-1-phenylethyl]ester (II: R¹ = R² = R³ = R⁴ = R^{1'} = R^{2'}= R^{3'}= R^{4'} = H; Y = O; R = (S)-1-Phenylethyl)

0.76 g (3.04 mmol) 2,2'-Biphenylphosphorigsäureesterchlorid wurden bei Raumtemperatur in 100 ml abs. Toluol vorgelegt. Hierzu wurden 1.00 ml (0.73 g, 7.22 mmol) abs. Triethylamin und 0.37 ml (0.37g, 3.04 mmol) (*S*)-1-Phenylethanol bei Raumtemperatur pipettiert. Nach Rühren über Nacht wurde der ausgefallene farblose Feststoff wird über eine D4-Fritte abfiltriert und mit 5 ml abs. Toluol gewaschen. Das Filtrat wurde anschließend vollständig vom Lösungsmittel befreit. Man erhielt 1.00 g (2.97 mmol, 97.8%) Produkt als gelbes Öl. Analytik: ¹H-NMR (CDCl₃, 300 MHz): 7.39-7.16 (m) [11H], 6.90-6.75 (m) [2H], 5.38 (m) [1H], 1.51 (d) *J* = 6.5 Hz [3H]; ³¹P-NMR (CDCl₃, 121 MHz): 142.870 (s); MS (EI, Verdampfungstemperatur 65°C): m/z = 336 (13.53%), 232 (99.23%), 168 (15.83%), 139 (7.90%), 105 (100%), 79 (9.82%); EA: C: 71.51% (ber. 71.42%), H: 5.16% (ber. 5.09%), P: 9.14% (ber. 9.21%).

### Beispiel 9. Synthese von (R)-2,2'-Binaphthylphosphorigsäurephenylmonothioester (I: R¹ = R² = R³ = R⁴ = R⁵ = R⁶ = R^{1'} = R^{2'} = R^{3'} = R^{4'} = R^{5'} = R^{6'} = H; Y = S; R = Phenyl)

0.94 g (2.69 mmol) (*R*)-2,2'-Binaphthylphosphorigsäureesterchlorid wurden bei Raumtemperatur in 100 ml abs. Toluol vorgelegt. Hierzu wurden 0.38 ml (0.28 g, 2.73 mmol) abs. Triethylamin und 0.28 ml (0.30 g, 2.69 mmol) Thiophenol bei Raumtemperatur pipettiert. Nach Rühren über Nacht wurde der ausgefallene farblose Feststoff wird über eine D4-Fritte abfiltriert und mit 5 ml abs. Toluol gewaschen. Das Filtrat wurde anschließend vollständig vom Lösungsmittel befreit. Man erhielt 0.99 g (2.34 mmol, 87.0%) Produkt als gelbes Pulver. Analytik: ¹H-NMR (CDCl₃, 300 MHz): 7.95-7.86 [4H], 7.57-7.20 [13H]; ³¹P-NMR (CDCl₃, 121 MHz): 217.733 (s); MS (EI, Verdampfungstemperatur 160°C): m/z = 424 (37.93%), 315 (100%), 268 (67.93%), 252 (26.63%), 239 (41.16%), 110 (10.85%); EA: C: 73.15% (ber. 73.57%), H: 3.88% (ber. 4.04%), P: 7.15% (ber. 7.30%).

### Darstellung von Metallkomplexen

### Beispiel 10. Synthese von (η²,η²-Cycloocta-1,5-dien)[(S)-2,2'-binaphthylphosphorigsäure-(R)-(1-phenyl)ethylester]-rhodium(I)-tetrafluoroborat

0.244 g (0.56 mmol) [(S)-2,2'-Binaphthylphosphorigsäure-(R)-(1-phenyl)ethylester] und 0.277 g (0.56 mmol) Bis-(1,5-cyclooctadien)-rhodium(I)-tetrafluoroborat wurden bei Raumtemperatur in 30 ml abs. Dichlormethan 20 h gerührt. Die orangefarbene Lösung wurde anschließend vollständig vom Lösungsmittel befreit. Der Rückstand wurde in 20 ml abs. Diethylether aufgenommen und der ausgefallene Feststoff über eine D4 Fritte abfiltriert. Man erhielt 0.21 g (0.29 mmol, 51.7%) Produkt als rotoranges Pulver. Analytik: ³¹P-NMR (CD₂Cl₂, 121 MHz): 137.520 (d, ¹*J*_{RhP} = 247 Hz); EA: C: 59.80% (ber. 58.88%), H: 5.18% (ber. 4.53%), P 4.18% (ber. 4.21%), Rh 14.16% (ber. 14.01%).

### Beispiel 11. Synthese von (η²,η²-Cycloocta-1,5-dien)-bis[(S)-2,2'-binaphthylphosphorigsäure[(S)-2-butyl]ester]-rhodium(I)-tetrafluoroborat ([Rh(cod)(I)_{2]}BF₄ mit I:R¹=R²=R³=R⁴=R⁵=R⁶=R^{1'}=R^{2'}=R^{3'}=R^{4'}=R^{5'}=R^{6'}=H;Y=O; R = (S)-2-Butyl; cod = η²,η²-Cycloocta-1,5-dien)

0.11 g (0.26 mmol) Rh(cod)₂BF₄ wurden in 15 ml Dichlormethan bei -78 °C vorgelegt und langsam mit einer Lösung von 0.20 g (0.52 mmol) (*S*)-2,2'-Binaphthylphosphorigsäure[(S)-2-bütyl]ester in 5 ml Dichlormethan versetzt. Die Lösung wurde über 20 h langsam auf Raumtemperatur erwärmt und das Lösungsmittel anschließend im Vakuum entfernt. Das verbleibende gelbe Öl wurde in 5 ml Pentan suspendiert und 24 h gerührt. Es bildete sich ein gelber Feststoff. Das Lösungsmittel wurde abfiltriert und der Feststoff zweimal mit je 5 ml Pentan gewaschen und im Vakuum getrocknet. Man erhielt 0.26 g (0.24 mmol, 92.3%) Produkt als gelbes Pulver. Analytik: ¹H-NMR (CD₂Cl₂, 300 MHz): 8.11-7.13 (m) [24H], 5.70 (m) [2H], 5.23 (m) [2H], 4.35 (m) [4H], 2.19-1.21 (m) [10H], 1.15 (d) *J* = 6.1 Hz [6H], 0.91 (t) *J* = 7.4 Hz [6H]; ³¹P-NMR (CD₂Cl₂, 121 MHz): 119.983 (d) *J =* 257.6 Hz; MS (ESI/pos. in CH₂Cl₂): m/z = 987 (100%) [M-BF₄], 877 (19.89%); EA: C: 62.28% (ber. 62.59%), H: 5.14% (ber. 5.06%), P: 5.74% (ber. 5.76%).

### Hydrierungen

### Allgemeine Vorschrift zur Hydrierung mit präformiertem Katalysator

1.0 ml einer 1 mM Lösung des angegebenen Katalysators in Dichlormethan wurde in einem Rundkolben mit Seithahn vorgelegt. Hierzu wurden 9.0 ml einer 0.11 M Substratlösung in Dichlormethan gegeben. Die Lösung wurde nun mit Wasserstoff gesättigt und unter 1.3 bar Wasserstoffdruck für die angegebene Zeit t bei Raumtemperatur gerührt. 2 ml der so erhaltenen Lösung wurden über 150 mg Silica (70-230 mesh, Aktivitätsstufe I) filtriert und gaschromatographisch analysiert.

### Allgemeine Vorschrift zur Hydrierung mit in situ hergestelltem Katalysator

0.5 ml einer 2 mM Lösung von Rh(cod)₂BF₄ in Dichlormethan wurde in einem Rundkolben mit Seithahn vorgelegt. Hierzu wurden 0.5 ml einer Lösung der angegebenen Konzentration c des angegebenen Liganden L und anschließend 9.0 ml einer 0.11 M Substratlösung in Dichlormethan gegeben. Die Lösung wurde nun mit Wasserstoff gesättigt und unter 1.3 bar. Wasserstoffdruck für die angegebene Zeit-t bei Raumtemperatur gerührt. 2 ml der so erhaltenen Lösung wurden über 150 mg Silica (70-230 mesh, Aktivitätsstufe 1) filtriert und gaschromatographisch analysiert.

### Enantioselektive Hydrierung von Dimethylitaconat mit präformiertem Katalysator

### Beispiel 12-13.

Die Beispiele 12-13 beschreiben die Hydrierung des Substrates Dimethylitaconat zu 2-Methylbernsteinsäuredimethylester nach der "Allgemeinen Vorschrift zur Hydrierung mit präformiertem Katalysator". Die genauen Reaktionsbedingungen sowie die erzielten Umsätze und Enantioselektivitäten sind in der Tabelle 1 angegeben.

**Tabelle 1.**

| **Bsp. Katalysator^{[a]} [Rh(cod)(L)₂]BF₄** | | | **Zeit t** | **Umsatz** | **ee** |
|---|---|---|---|---|---|
| | Konfiguration von L | Rest R | h | in %^{[b]} | in % |
| **12.** | *(S)* | (S)-CH(CH₃)CH₂CH₃ | 20 | 100 | 96.8(*S*) |
| **13.** | (*S*) | (R)-CH(CH₃)C₆H₅ | 20 | 100 | 94.0 (*S*) |

| | | | | | |
|---|---|---|---|---|---|
| [a] Es handelt sich jeweils um einen Katalysator [Rh(cod)(L)₂]BF₄ mit Liganden L der Struktur I mit R¹ = R² = R³ = R⁴ = R⁵ = R⁶ = R^{1'} = R^{2'}= R^{3'} = R⁴' = R^{5'} = R⁶' = H und Y=O. [b] Falls gaschromatographisch kein Edukt mehr nachzuweisen war, ist als Umsatz 100% angegeben. | | | | | |

### Enantioselektive Hydrierung von Dimethylitaconat mit in situ hergestelltem Katalysator

### Beispiele 14-55.

Die Beispiele 14-55 beschreiben die Hydrierung des Substrates Dimethylitaconat zu 2-Methylbernsteinsäuredimethylester nach der "Allgemeinen Vorschrift zur, Hydrierung mit *in situ* hergestelltem Katalysator". Die genauen Reaktionsbedingungen sowie die erzielten Umsätze und Enantioselektivitäten sind in den Tabelle 2 bis 4 angegeben.

**Tabelle 2.**

| **Bsp. Ligand L^{[a]}** | | | | **Zeit t** | **Umsatz** | **ee** |
|---|---|---|---|---|---|---|
| | Konfig. | Rest R | Konz. c | h | in %^{[b]} | in % |
| **14.** | *(S)* | CH₃ | 2 mM | 20 | 100 | 89.2 (*S*) |
| **15.** | *(S)* | CH₃ | 4 mM | 3 | 100 | 83.2 (*S*) |
| **16.** | *(S)* | CH(CH₃)₂ | 2 mM | 20 | 100 | 97.6 (*S*) |
| **17.** | *(S)* | CH(CH₃)₂ | 4 mM | 3 | 100 | 97.0 (*S*) |
| **18.** | *(S)* | (*rac*)-CH(CH₃)C₆H₅ | 2 mM | 20 | 100 | 98.8 (*S*) |
| **19.** | *(S)* | (*rac*)-CH(CH₃)C₆H₅ | 4 mM | 3 | 100 | 98.6 (*S*) |
| **20**.^{[c]} | (*S*) | (*R*)-CH(CH₃)C₆H₅ | 2 mM | 20 | 100 | 99.2 (*S*) |
| **21.** | (*S*) | (*R*)-CH(CH₃)C₆H₅ | 4 mM | 3 | 100 | 98.8 (*S*) |
| **22.** | (*S*) | (S)-CH(CH₃)C₆H₅ | 2 mM | 20 | 100 | 98.2 (*S*) |
| **23**. | (*S*) | (S)-CH(CH₃)C₆H₅ | 4 mM | 3 | 100 | 96.8 (*S*) |
| **24**. | (*R*) | (R)-CH(CH₃)C₆H₅ | 4 mM | 3 | 100 | 97.8 (*R*) |
| **25.** | (*R*) | (R)-CH(CH₃)C₆H₅ | 2mM | 20 | 100 | 96.6 (*R*) |
| **26.** | (*R*) | (S)-CH(CH₃)C₆H₅ | 4 mM | 3 | 100 | 99.4 (*R*) |
| **27.** | (*R*) | (S)-CH(CH₃)C₆H₅ | 2 mM | 20 | 100 | 96.8 (*R*) |
| **28**. | (*S*) | CH(C₆H₅)₂ | 2 mM | 20 | 100 | 92.0 (*S*) |
| **29**. | (*S*) | C₆H₅ | 2 mM | 20 | 100 | 96.6 (*S*) |
| **30.** | (*S*) | C₆H₅ | 4 mM | 3 | 100 | 97.8 (*S*) |
| **31**. | (*S*) | CH(CF₃)₂ | 4 mM | 20 | 55.0 | 72.0 (*S*) |
| **32**. | (*S*) | (CH₂)₄CH₃ | 4 mM | 3 | 100 | 94.2 (*S*) |
| **33**. | (*S*) | CH(CH₂CH₂)₂ | 4 mM | 3 | 100 | 97.4 (*S*) |
| **34**. | (*S*) | CH(CH₂)₄ (Cyclopentyl) | 4 mM | 3 | 100 | 99.0 (*S*) |
| **35**. | *(S)* | Fluorenyl | 4 mM | 3 | 100 | 96.0 (S) |
| **36**. | *(S)* | C(CH₃)₃ | 4 mM | 3 | 100 | 91.4 (S) |
| **37.** | *(S)* | Naphthyl | 4 mM | 3 | 100 | 87.0 (S) |
| **38**. | *(S)* | (-)-Menthyl | 4 mM | 3 | 100 | 94.8 (S) |
| **39.** | *(S)* | (-)-Menthyl | 4 mM | 3 | 100 | 91.0 (S) |
| **40**. | *(S)* | C₆H₄N(CH₃)₂ (3-N,N- | 4 mM | 20 | 51.4 | 82.2 (S) |
| | | Dimethylaminophenyl) | | | | |
| **41.** | *(S)* | (CH₂)₂N(CH(CH₃)₂)₂ (N,N*-* | 4 mM | 20 | 100 | 84.6 (S) |
| | | Di-iso-propylaminoethyl) | | | | |
| **42**. | *(S)* | CH₂C₆H₅ | 4 mM | 20 | 100 | 98.2 (S) |
| **43.** | (*S*) | (*S*)-CH(CH₃)CH₂CH₃ | 4 mM | 20 | 100 | 98.5 (*S*) |
| **44.** | (*R*) | (*S*)-CH(CH₃)CH₂CH₃ | 4 mM | 20 | 100 | 98.6 *(R)* |
| **45.** | (*S*) | (*S*)-CHCH(CH₃)CH₂CH₃ | 4 mM | 20 | 100 | 64.4 (*S*) |
| **46.** | (*R*) | (*S*)-CHCH(CH₃)CH₂CH₃ | 4 mM | 20 | 100 | 97.4 (*R*) |
| **47.** | *(R*) | CH₂C(CH₃)₃ | 4 mM | 20 | 100 | 98.6 *(R)* |
| **48.** | (*S*) | (*S*)-CH(CH₃)CO₂CH₂CH₃ | 4 mM | 20 | 100 | 91.0 (*S*) |
| **49.** | (*R*) | (*S*)-CH(CH₃)CO₂CH₂CH₃ | 4 mM | 20 | 84.0 | 67.8 *(R)* |
| **50.** | (*S*) | CH₂CH₂OCH₃ | 4 mM | 3 | 100 | 94.4 (*S*) |
| **51.** | *(R)* | CH₂CH₂OCH₃ | 4 mM | 20 | 100 | 95.1 *(R)* |

| | | | | | | |
|---|---|---|---|---|---|---|
| [a] Es handelt sich jeweils um einen Liganden der Struktur I mit R¹ = R² = R³ = R⁴ = R⁵ = R⁶ =R^{1'} =R^{2'} =R^{3'} =R^{4'} =R^{5'} =R^{6'} = H und Y = O. [b] Falls gaschromatographisch kein Edukt mehr nachzuweisen war, ist als Umsatz 100% angegeben. [c] Bei Verwendung von jeweils nur 0.1 ml bzw. jeweils nur 0.2 ml 2 mM Rh(cod)₂BF₄-Lösung und 2 mM Ligandlösung wird nach 20 h bei quantitativer Hydrierung ein Enantiomerenüberschuss (ee) von jeweils 99.40% zugunsten des S- konfigurierten Produkts gemessen. | | | | | | |

**Tabelle 3.**

| **Bsp. Ligand L^{[a]}** | | | | **Zeit t** | **Umsatz** | **ee** |
|---|---|---|---|---|---|---|
| | Reste R³ u.R^{3'} | Rest R | Konz. c | h | in %^{[b]} | in % |
| **52.** | H | (*S*)-CH(CH₃)CH₂CH₃ | 4 mM | 20 | 100 | 11.8 (*S*) |
| **53.** | H | (*S*)-CH(CH₃)C₆H₅ | 4 mM | 20 | 100 | 30.0 (*R*) |
| **54**. | CH(CH₃)₂ | (*S*)-CH(CH₃)C₆H₅ | 4 mM | 20 | 100 | 15.6 (*R*) |

| | | | | | | |
|---|---|---|---|---|---|---|
| [a] Es handelt sich jeweils um einen Liganden der Struktur II mit R¹ =R² = R⁴ = R^{1'}= R^{2'}=R^{4'}= H und Y = O. [b] Falls gaschromatographisch kein Edukt mehr nachzuweisen war, ist als Umsatz 100% angegeben. | | | | | | |

**Tabelle 4.**

| **Bsp. Ligand L^{[a]}** | | | | **Zeit** | **Umsatz** | **ee** |
|---|---|---|---|---|---|---|
| | Konfig. | Rest R | Konz. c | h | in % | in % |
| **55.** | (*R*) | C₆H₅ | 4 mM | 20 | 55.2 | 66.6 (*R*) |

| | | | | | | |
|---|---|---|---|---|---|---|
| [a] Es handelt sich um einen Liganden der Struktur I mit R¹ = R² = R³ = R⁴ = R⁵ = R⁶ = R^{1'} = R^{2'}= R^{3'} = R^{4'} = R^{5'} = R^{6'} = H und Y = S. | | | | | | |

### Enantioselektive Hydrierung von 2-Acetamidoacrylsäuremethylester mit präformiertem Katalysator

### Beispiel 56.

Das Beispiel 56 beschreibt die Hydrierung des Substrates 2-Acetamidoacrylsäuremethylester zu N-Acetylalaninmethylester nach der "Allgemeinen Vorschrift zur Hydrierung mit präformiertem Katalysator". Die genauen Reaktionsbedingungen sowie die erzielten Umsätze und Enantioselektivitäten sind in der Tabelle 5 angegeben.

**Tabelle 5.**

| **Bsp. Katalysator^{[a]} [Rh(cod)(L)]BF₄₂** | | | **Zeit t** | **Umsatz** | **ee** |
|---|---|---|---|---|---|
| | Konfiguration von L | Rest R | h | in %^{[b]} | in % |
| **56.** | (*S*) | (*S*)-CH(CH₃)CH₂CH₃ | 20 | 100 | 94.8 *(R)* |

| | | | | | |
|---|---|---|---|---|---|
| [a] Es handelt sich um einen Katalysator [Rh(cod)(L)₂]BF₄ mit Liganden L der Struktur I mit R¹=R²=R³=R⁴=R⁵=R⁶=R^{1'}=R^{2'}=R^{3'}=R^{4'}=R^{5'}=R^{6'}=H und Y=O. [b] Falls gaschromatographisch kein Edukt mehr nachzuweisen war, ist als Umsatz 100% angegeben. | | | | | |

### Enantioselektive Hydrierung von 2-Acetamidoacrylsäuremethylester mit in situ hergestelltem Katalysator

### Beispiele 57-80.

Die Beispiele 57-80 beschreiben die Hydrierung des Substrates 2-Acetamidoacrylsäuremethylester zu N-Acetylalaninmethylester nach der "Allgemeinen. Vorschrift zur Hydrierung mit *in situ* hergestelltem Katalysator". Die genauen Reaktionsbedingungen sowie die erzielten Umsätze und Enantioselektivitäten sind in den Tabelle 6 und 7 angegeben.

**Tabelle 6.**

| **Bsp. Ligand L^{[a]}** | | | | **Zeit t** | **Umsatz** | **ee** |
|---|---|---|---|---|---|---|
| | Konfig. | Rest R | Konz. c | h | in %^{[b]} | in % |
| **57.** | (*S*) | CH₃ | 2 mM | 20 | 100 | 72.8 (*R*) |
| **58.** | (*S*) | CH(CH₃)₂ | 2 mM | 20 | 100 | 94.8 (*R*) |
| **59**. | (*S*) | CH(CH₃)₂ | 4 mM | 3 | 100 | 93.0 *(R)* |
| **60**. | (*S*) | *(rac)-CH(CH₃)C₆H₅* | 2 mM | 20 | 100 | 99.9 *(R)* |
| **61.** | (*S*) | *(rac)-CH(CH₃)C₆H₅* | 4 mM | 3 | 100 | 92.4 *(R)* |
| **62.** | (*S*) | (R)-CH(CH₃)C₆H₅ | 2 mM | 20 | 100 | 95.5 (*R*) |
| **63**. | (*S*) | (*R*)-CH(CH₃)C₆H₅ | 4 mM | 3 | 100 | 95.6 (*R*) |
| **64.** | (*S*) | (S)-CH(CH₃)C₆Hₛ | 2 mM | 20 | 100 | 93.3 (*R*) |
| **65**. | (*S*) | C₆H₅ | 2 mM | 20 | 100 | 80.6 (*R*) |
| **66.** | (*S*) | CH(CH₂CH₃)₂ | 4 mM | 3 | 100 | 94.8 (*R*) |
| **67.** | (*S*) | CH(CH₂)₄ (Cyclopentyl) | 4 mM | 3 | 100 | 96.6 (*R*) |
| **68.** | (*S*) | C(CH₃)₃ | 4 mM | 3 | 100 | 95.4 (*R*) |
| **69.** | (*S*) | CH₂(C₆H₅) | 4 mM | 20 | 100 | 88.9 (*R*) |
| **70.** | (*S*) | (*S*)-CH(CH₃)CH₂CH₃ | 4 mM | 20 | 100 | 95.6 (*R*) |
| **71.** | (*R*) | (*S*)-CH(CH₃)CH₂CH₃ | 4 mM | 20 | 100 | 95.6 (*S*) |
| **72.** | (*S*) | (*S*)-CHCH(CH₃)CH₂CH₃ | 4 mM | 20 | 100 | 90.6 (*R*) |
| **73.** | (*R*) | (*S*)-CHCH(CH₃)CH₂CH₃ | 4 mM | 20 | 100 | 92.8 (*S*) |
| **74.** | (*R*) | CH₂C(CH₃)₃ | 4 mM | 20 | 100 | 93.0 (*S*) |
| **75.** | (*S*) | (*S*)-CH(CH₃)CO₂CH₂CH₃ | 4 mM | 20 | 100 | 87.8 (*R*) |
| **76.** | (*R*) | (*S*)-CH(CH₃)CO₂CH₂CH₃ | 4 mM | 20 | 84.0 | 73.0 (*S*) |
| **77.** | *(R)* | CH₂CH₂OCH₃ | 4 mM | 20 | 100 | 88.2 (*S*) |

| | | | | | | |
|---|---|---|---|---|---|---|
| [a] Es handelt sich jeweils um einen Liganden der Struktur I mit R¹ = R² = R³ = R⁴ = R⁵ = R⁶=R^{1'}=R^{2'}=R^{3'}=R^{4'}=R^{5'}=R^{6'}=H und Y=O. [b] Falls gaschromatographisch kein Edukt mehr nachzuweisen war, ist als Umsatz 100% angegeben. | | | | | | |

**Tabelle 7.**

| **Bsp. Ligand L^{[a]}** | | | | **Zeit t** | **Umsatz** | **ee** |
|---|---|---|---|---|---|---|
| | Reste R³ u. R^{3'} | Rest R | Konz. c | h | in %^{[b]} | in % |
| **78.** | H | (*S*)-CH(CH₃)CH₂CH₃ | 4 mM | 20 | 100 | 10.0 (R) |
| **79.** | H | (*S*)-CH(CH₃)C₆H₅ | 4 mM | 20 | 100 | 18.2 (R) |
| **80.** | CH(CH₃)₂ | (*S*)-CH(CH₃)C₆H₅ | 4 mM | 20 | 100 | 22.8 (R) |

| | | | | | | |
|---|---|---|---|---|---|---|
| [a] Es handelt sich jeweils um einen Liganden der Struktur II mit R¹ = R² = R⁴ = R^{1'} = R^{2'}= R^{4'}= H und Y = O. [b] Falls gaschromatographisch kein Edukt mehr nachzuweisen war, ist als Umsatz 100% angegeben. | | | | | | |

Enantioselektive Hydrierung von (Z)-2-Acetamidozimtsäuremethylester mit ***in situ* hergestelltem Katalysator**

### Beispiele 81-97.

Die Beispiele 81-97. beschreiben die Hydrierung des Substrates (Z)-2-Acetamidozimtsäuremethylester zu N-Acetylphenylalaninmethylester nach der "Allgemeinen Vorschrift zur Hydrierung mit *in situ* hergestelltem Katalysator". Die genauen Reaktionsbedingungen sowie die erzielten Umsätze und Enantioselektivitäten sind in der Tabelle 8 angegeben.

**Tabelle 8.**

| **Bsp.** | **Ligand L^{[a]}** | | | **Zeit t** | **Umsatz** | **ee** |
|---|---|---|---|---|---|---|
| | Konfig. | Rest R | Konz. c | h | in %^{[b]} | in % |
| **81.** | (*S*) | CH₃ | 4 mM | 20 | 100 | 53.0 (*R*) |
| **82.** | (*S*) | CH₃ | 2 mM | 20 | 100 | 55.8 (*R*) |
| **83.** | (*S*) | CH(CH₃)₂ | 4 mM | 3 | 100 | 87.2 (*R*) |
| **84**. | (*S*) | CH(CH₃)₂ | 2 mM | 20 | 100 | 88.2 (*R*) |
| **85.** | (*S*) | (*S*)-CH(CH₃)C₆H₅ | 4 mM | 3 | 98.9 | 85.0 (*R*) |
| **86.** | *(S)* | (*S*)-CH(CH₃)C₆H₅ | 2 mM | 20 | 91.7 | 84.8 *(R)* |
| **87.** | *(S)* | (*R*)-CH(CH₃)C₆H₅ | 4 mM | 3 | 97.2 | 88.6 *(R)* |
| **88.** | *(S)* | (*R*)-CH(CH₃)C₆H₅ | 2 mM | 20 | 100 | 91.7 (*R*) |
| **89.** | *(S)* | (*rac*)-CH(CH₃)C₆H₅ | 2 mM | 20 | 100 | 87.8 *(R)* |
| **90.** | *(R)* | (*S*)-CH(CH₃)C₆H₅ | 2 mM | 20 | 100 | 92.2 (*S*) |
| **91.** | *(R)* | (*R*)-CH(CH₃)C₆H₅ | 2 mM | 20 | 100 | 86.6 (*S*) |
| **92.** | *(S)* | C₆H₅ | 2 mM | 20 | 69.5 | 68.4 (*R*) |
| **93.** | *(S)* | CH(CH₂CH₃)₂ | 4 mM | 3 | 100 | 87.1 (*R*) |
| **94.** | *(S)* | CH(CH₂)₄ (Cyclopentyl) | 4 mM | 3 | 94.2 | 88.5 *(R)* |
| **95.** | *(S)* | C(CH₃)₃ | 4 mM | 3 | 100 | 82.8 *(R)* |
| **96**. | *(S)* | (-)-Menthyl | 4 mM | 3 | 100 | 44.9 *(R)* |
| **97**. | *(S)* | (+)-Menthyl | 4 mM | 3 | 96.0 | 53.0 *(R)* |

| | | | | | | |
|---|---|---|---|---|---|---|
| [a] Es handelt sich jeweils um einen Liganden der Struktur I mit R¹ = R² = R³ = R⁴ = R⁵ = R⁶=R^{1'}=R^{2'}=R^{3'}=R^{4'}=R^{5'}=R^{6'}=H und Y=O. [b] Falls gaschromatographisch kein Edukt mehr nachzuweisen war, ist als Umsatz 100% angegeben. | | | | | | |

## Patentansprüche

1. Verfahren zur asymmetrischen Übergangsmetall-katalysierten Hydrierung, von prochiralen Olefinen, Ketonen oder Ketiminen, in welchem die Katalysatoren chirale Monophosphite oder deren Monothioderivate als Liganden aufweisen, **dadurch gekennzeichnet dass** die Liganden ausgewählt sind aus den Liganden I - IV 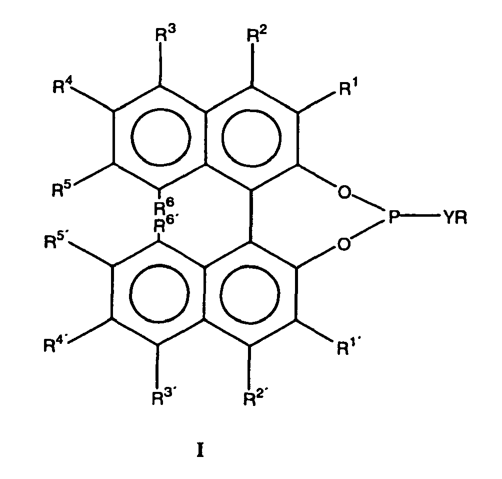 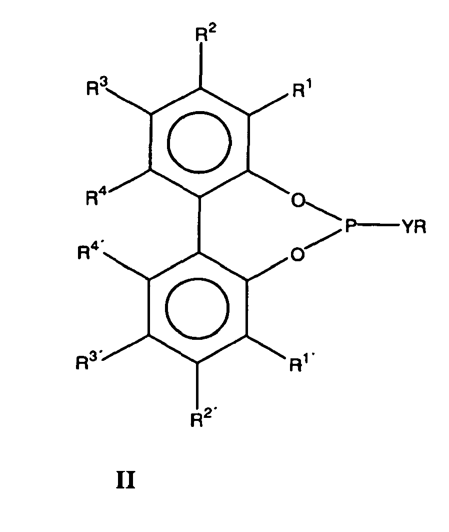 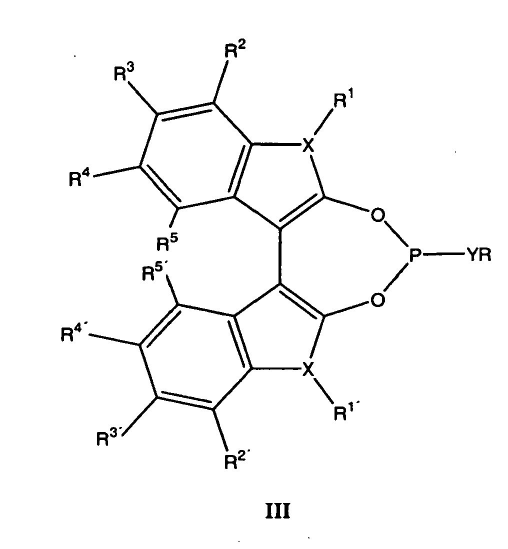 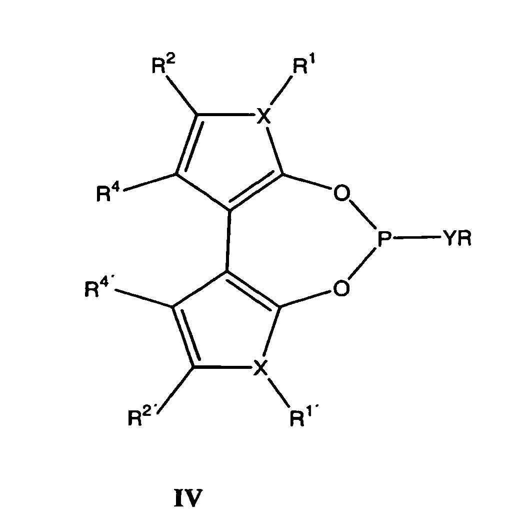 wobei Y für Sauerstoff oder Schwefel steht und der Rest R aus der Reihe Wasserstoff, gesättigte und ungesättigte lineare und verzweigte C₁-C₅₀ Alkyl-, C₁-C₅₀ Aryl- und Heteroarylgruppen ist
und die Reste R¹, R², R³, R⁴, R⁵, R⁶, R^{1'}, R^{2'}, R^{3'}, R^{4'}, R^{5'} und R^{6'} unabhängig voneinander aus der Reihe Wasserstoff, Halogen, gesättigte und ungesättigte lineare und verzweigte C₁-C₅₀ Alkyl-, C₁-C₅₀ Aryl-, C₁-C₅₀ Heteroaryl-, Alkinyl-, Silyl-, Nitro-, Nitril-, Ester-, Carboxyl-, Carbonyl-, Amid-, Amin-, Hydroxy-, Alkoxy-, Sulfid- und Selenidgruppen sind,
wobei R, R¹, R², R³, R⁴, R⁵, R⁶, R^{1'}, R^{2'}, R^{3'}, R^{4'}, R^{5'} und R^{6'} unabhängig voneinander ihrerseits weitere Substituenten tragen oder funktionalisiert sein können,
wobei einzelne oder mehrere Kohlenstoffatome des Binaphthylgerüstes oder des Biphenylgerüstes der Strukturen I bzw. **II** unabhängig voneinander durch die Heteroatome Si, O, N oder S ersetzt sein können,
wobei X Sauerstoff, Schwefel oder Stickstoff sein kann, wobei für X = O oder X = S die Substituenten R¹ und R^{1'} entfallen.

2. Verfahren nach Anspruch 1, wobei ein Ligand der Formel **I** oder **III** eingesetzt wird.

3. Verfahren nach Anspruch 1, wobei ein Ligand der Formel **II** oder **IV** eingesetzt wird,
wobei R⁴ = R^{4'} = H ist und R chiral ist.

4. Verfahren nach Anspruch 1, wobei ein Ligand der Formel **II** oder **IV** eingesetzt wird,
wobei R⁴ ≠ H und R^{4'} ≠ H ist.

5. Verfahren nach Ansprüchen 1-4, wobei die Übergangsmetall-Katalysatoren Übergangsmetalle der Gruppen VIII und Ib des Periodensystems enthalten.

6. Verfahren nach Ansprüchen 1 - 5, wobei als Katalysator einer der Komplexe VI - XXIX eingesetzt wird, in denen Z ein Anion aus der Reihe BF₄⁻, BAr₄⁻ , SbF₆⁻ und PF₆⁻ ist, wobei Ar = Phenyl oder ist.

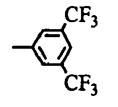

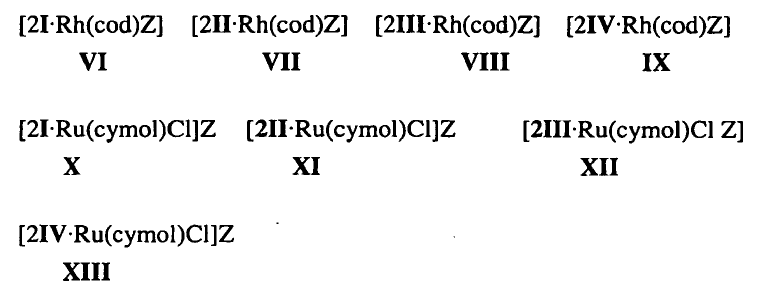

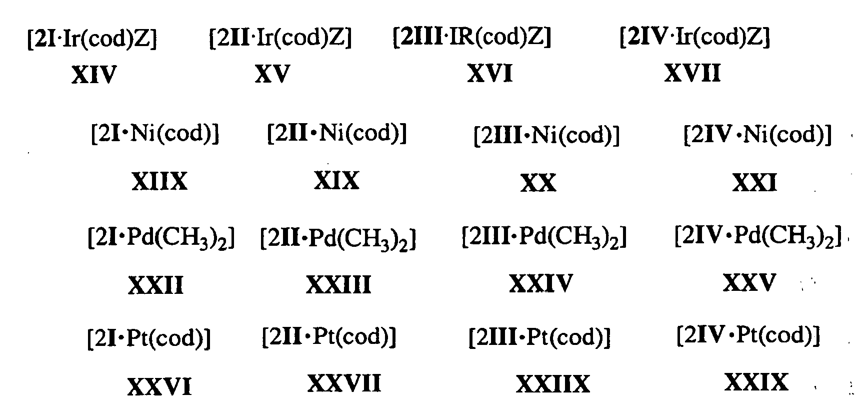

## Claims

1. Process for the asymmetrical transition-metal-catalysed hydrogenation of prochiral olefins, ketones or ketimines wherein the catalysts have chiral monophosphites or their monothio derivatives as ligands, **characterized in that** the ligands are selected from the ligands **I-IV** 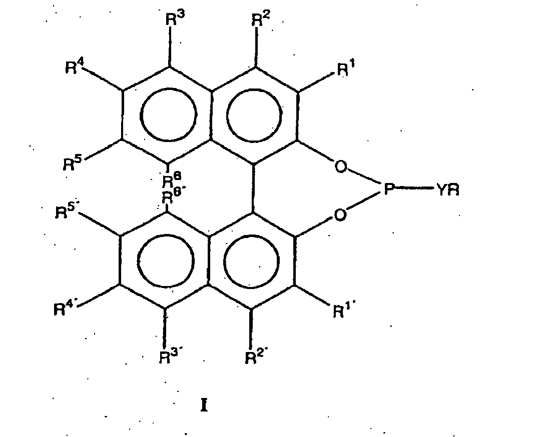 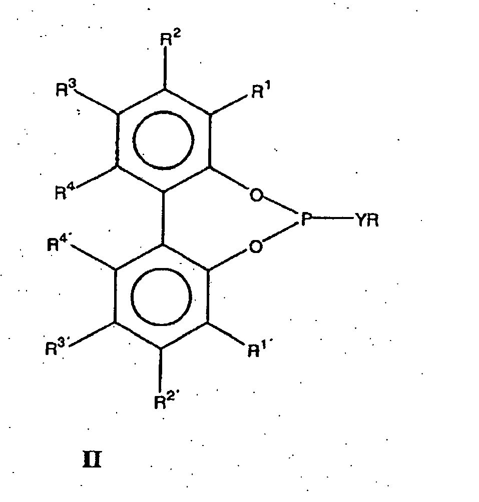 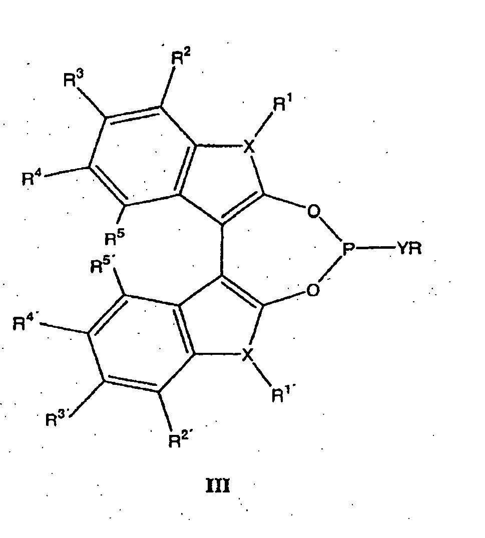 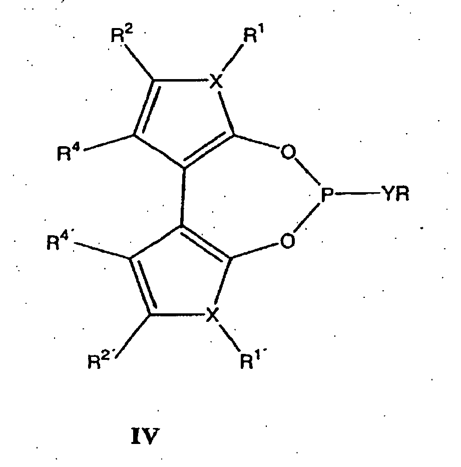 where Y represents oxygen or sulphur and the R radical is from the group consisting of hydrogen, saturated and unsaturated linear and branched C₁-C₅₀ alkyl, C₁-C₅₀ aryl and heteroaryl groups
and the R¹, R², R³, R⁴, R⁵, R⁶, R^{1'}, R^{2'}, R^{3'}, R^{4'}, R^{5'} and R^{6'} radicals are independently from the group consisting of hydrogen, halogen, saturated and unsaturated linear and branched C₁-C₅₀ alkyl, C₁-C₅₀ aryl, C₁-C₅₀ heteroaryl, alkynyl, silyl, nitro, nitrile, ester, carboxyl, carbonyl, amide, amine, hydroxy, alkoxy, sulphide and selenide groups,
and R, R¹, R², R³, R⁴, R⁵, R⁶, R^{1'}, R^{2'}, R^{3'}, R^{4'}, R^{5'}, and R ⁶ may independently in turn bear further substituents or be functionalized,
individual or a plurality of carbon atoms of the binaphthyl scaffold or of the biphenyl scaffold of the structures I and II respectively may be independently replaced by the heteroatoms Si, O, N or S,
and X can be oxygen, sulphur or nitrogen, in which case the substituents R¹ and R^{1'} are omitted for X = O or X = S.

2. Process according to Claim 1, wherein a ligand of formula I or III is employed.

3. Process according to Claim 1, wherein a ligand of formula II or IV is employed, R⁴ = R^{4'} = H and R is chiral.

4. Process according to Claim 1, wherein a ligand of formula **II** or **IV** is employed, R⁴≠ H and R^{4'}≠ H.

5. Process according to Claims 1-4, wherein the transition metal catalysts contain transition metals of groups VIII and Ib of the Periodic Table.

6. Process according to Claims 1-5, wherein the catalyst employed is one of the complexes **VI-XXIX** in which Z is an anion from the group consisting of BF₄⁻, BAr₄⁻, SbF₆⁻ and PF₆⁻, where Ar = phenyl or is

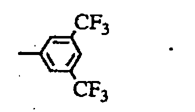

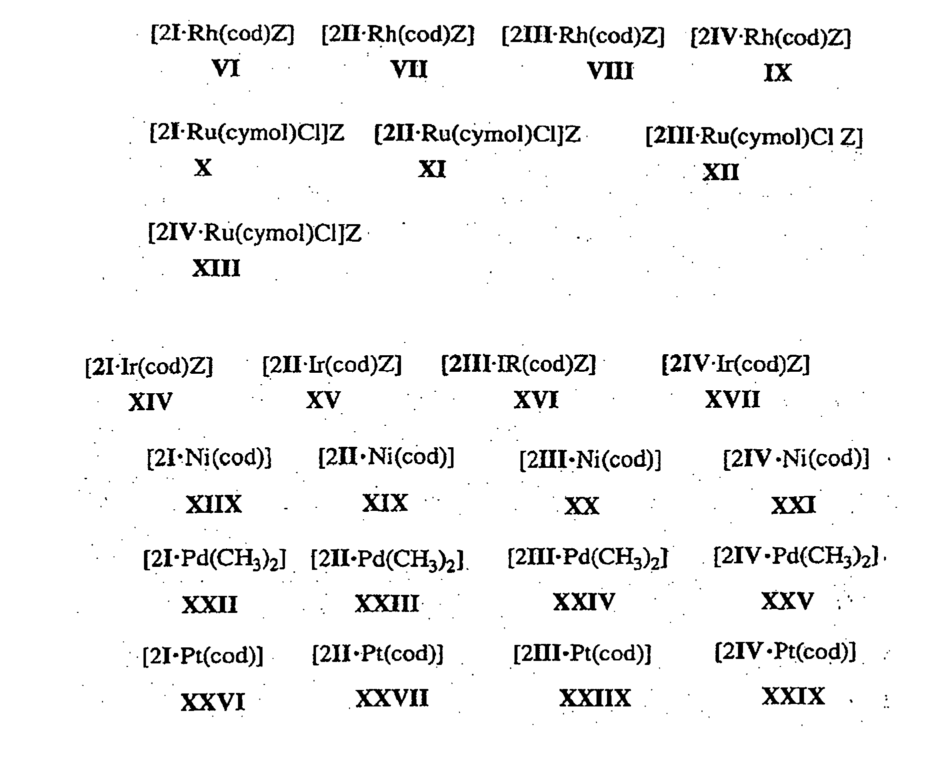

## Revendications

1. Procédé d'hydrogénation asymétrique, catalysée par un métal de transition, d'oléfines, de cétones ou de cétimines prochirales, dans lequel les catalyseurs comprennent des monophosphites chiraux ou leurs dérivés monothio en tant que ligands, **caractérisé en ce que** les ligands sont choisis parmi les ligands **I-IV** 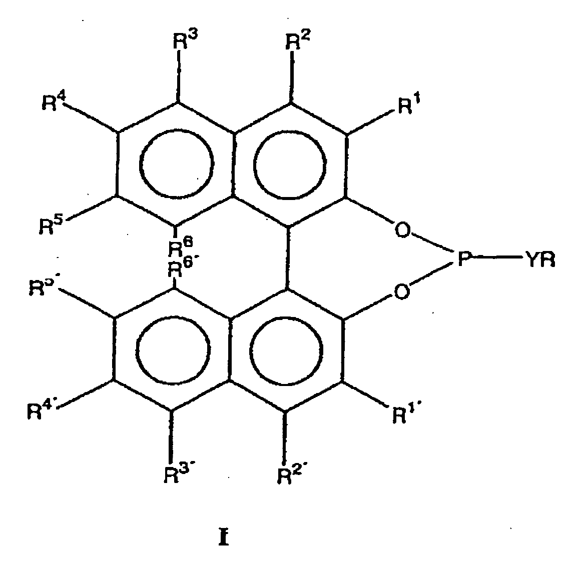 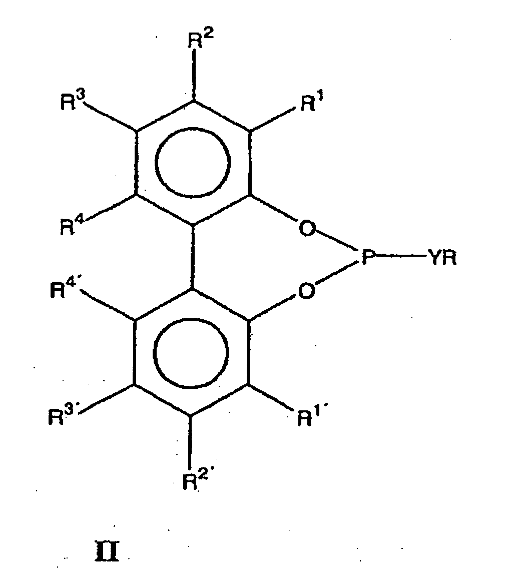 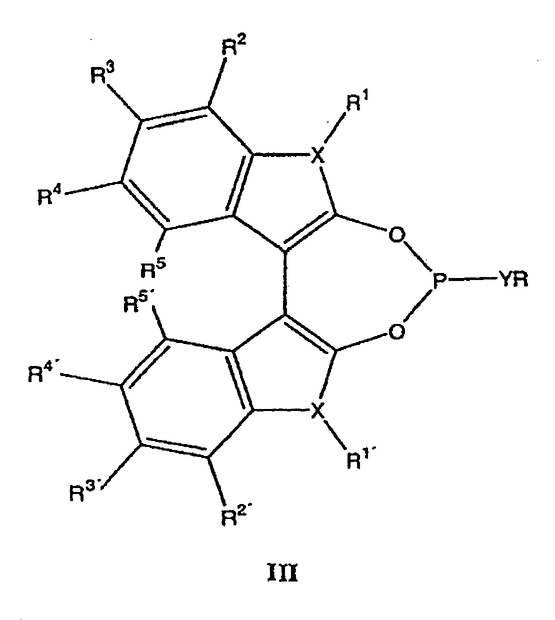 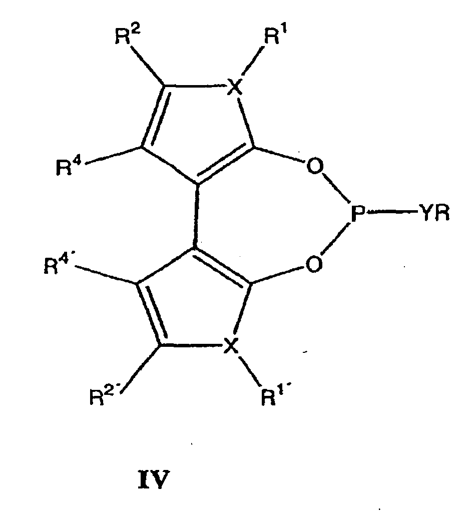 dans lesquels Y représente un atome d'oxygène ou un atome de soufre, et le radical R est parmi le groupe constitué d'un atome d'hydrogène, de groupes alkyle en C₁-C₅₀ saturés et insaturés, linéaires et ramifiés, de groupes aryle en C₁-C₅₀. et de groupes hétéroaryle,
et les radicaux R¹, R², R³, R⁴, R⁵, R⁶, R^{1'}, R^{2'}, R^{3'}, R^{4'}, R^{5'} et R^{6'}, indépendamment les uns des autres, sont parmi le groupe constitué d'un atome d'hydrogène, d'un atome d'halogène, de groupes alkyle en C₁-C₅₀ saturés et insaturés, linéaires et ramifiés, de groupes aryle en C₁-C₅₀, de groupes hétéroaryle en C₁-C₅₀, de groupes alcynyle, d'un groupe silyle, d'un groupe nitro, d'un groupe nitrile, de groupes ester, d'un groupe carboxyle, d'un groupe carbonyle, de groupes amide, de groupes amine, d'un groupe hydroxy, d'un groupe alcoxy, d'un groupe sulfure et d'un groupe séléniure,
où R, R¹, R², R³, R⁴, R⁵, R⁶, R^{1'}, R^{2'}, R^{3'}, R^{4'}, R^{5'} et R^{6'}, indépendamment les uns des autres, peuvent, à leur tour, renfermer des substituants supplémentaires ou être fonctionnalisés,
où des atomes de carbone individuels ou plusieurs atomes de carbone du squelette binaphtyle ou du squelette biphényle des structures I ou II peuvent, indépendamment les uns des autres, être remplacés par les hétéroatomes silicium, oxygène, azote ou soufre,
où X peut être un atome d'oxygène, un atome de soufre ou un atome d'azote, où pour X = 0 ou X = S, les substituants R¹ and R^{1'} sont supprimés.

2. Procédé selon la revendication 1, dans lequel un ligand de formule I ou III est utilisé.

3. Procédé selon la revendication 1, dans lequel un ligand de formule II ou IV est utilisé, dans laquelle R⁴ = R^{4'}= H et R est chiral.

4. Procédé selon la revendication 1, dans lequel un ligand de formule **II** ou **IV** est utilisé, dans laquelle R⁴≠ H et R^{4'}≠ H.

5. Procédé selon les revendications 1 à 4, dans lequel les catalyseurs à métaux de transition renferment des métaux de transition des groupes VIII et Ib du système périodique.

6. Procédé selon les revendications 1 à 5, dans lequel on utilise, en tant que catalyseur, l'un des complexes **VI-XXIX** dans lesquels Z est un anion parmi le groupe constitué de BF₄⁻, de BAr₄⁻, de SbF₆⁻ et de PF₆⁻, où Ar est un groupe phényle ou un groupe
